# EUROPÄISCHE PATENTSCHRIFT

(11) **EP 2 448 500 B1**
(45) Veröffentlichungstag und Bekanntmachung des Hinweises auf die Patenterteilung: **02.07.2014**
(21) Anmeldenummer: 10739484.3
(22) Anmeldetag: 16.06.2010
(51) Int. Cl.: A61B 17/15, A61B 17/16

(54) **VORRICHTUNG ZUR IN-SITU-FRÄSUNG VON GELENKFLÄCHEN**
DEVICE FOR IN SITU MILLING OF JOINT SURFACES
DISPOSITIF POUR LE FRAISAGE IN SITU DE SURFACES ARTICULAIRES

(30) Priorität: 30.06.2009 DE 102009031269
(43) Veröffentlichungstag der Anmeldung: 09.05.2012
(73) Patentinhaber: Universität Rostock, 18051 Rostock (DE)
(72) Erfinder: VASARHELYI, Attila, 04275 Leipzig (DE); HARTMANN, Rolf, 50745 Boras (DE)
(74) Vertreter: Volmer, Jochen
(86) Internationale Anmeldenummer: PCT/EP2010/003620
(87) Internationale Veröffentlichungsnummer: WO 2011/000476

(56) Entgegenhaltungen:
- EP-A1- 1 374 783
- WO-A1-01/66021

## Beschreibung

Die Erfindung betrifft eine Vorrichtung zur In-situ-Fräsung von Gelenkflächen, umfassend eine Fräsvorrichtung und eine Gelenkspannvorrichtung. Die Erfindung betrifft weiter eine Verwendung einer entsprechenden Vorrichtung sowie ein Verfahren zur In-situ-Fräsung von Gelenkflächen.

Eine Arthrose ist eine chronische degenerative Gelenkveränderung, ausgezeichnet durch Ab- und Umbauvorgänge des Gelenkknorpels. Eine Arthrose entsteht aufgrund eines Missverhältnisses zwischen Beanspruchung und Leistungsfähigkeit der entsprechenden Gelenkanteile. Die Arthrose kann durch angeborene oder erworbene Vorschädigungen verschlimmert werden. Durch Abnutzung und Verschleiß eines Gelenkknorpels wird dessen Funktion zunehmend eingeschränkt und führt unter anderem zu Bewegungs- und Belastungsschmerzen.

Etablierte konservative Therapien zur Linderung der durch die Gelenkbeeinträchtigung entstehenden Beschwerden versagen in schweren Fällen. In solchen Fällen wird die Operation des Gelenks mit einem künstlichen Gelenkersatz, einer Endoprothese, angestrebt. Bei den Endoprothesen kann zwischen sogenannten Oberflächenersatz-, Halb- und Totalendoprothesen unterschieden werden.

Abhängig von den anatomischen Gegebenheiten eines betroffenen Gelenks oder Gelenktyps werden bestimmte Knochen- und Knorpelanteile entfernt, um diese danach durch Prothesenmaterial zu ersetzen. Dabei muss der Operateur das nötige Ausmaß der zu entfernenden Skelettstrukturen abschätzen, um nach Einsetzen der Prothesenkomponenten ein biomechanisch einwandfrei funktionierendes Gelenk zu erhalten. Dies erweist sich besonders bei scharnierartig funktionierenden Gelenken, z.B. dem Kniegelenk, dem EIlenbogengelenk oder dem oberen Sprunggelenk als schwer kalkulierbar.

Es ist ein Ziel bei solchen Operationen, die Prothese so zu positionieren, dass die ursprünglich bestehende Achsenkinematik und Ligamentführung des betroffenen Gelenks wiederhergestellt wird, um sowohl eine ausreichende Beweglichkeit des Gelenks als auch eine gute Stabilität in den unterschiedlichen einzunehmenden Stellungen des Gelenks zu ermöglichen. Wenn diese Voraussetzungen nicht erfüllt sind, wird die Funktion des Gelenks beeinträchtigt. Dies führt außerdem aufgrund erhöhter Scherkräfte auf das Prothesenmaterial zu erhöhtem Abrieb und gegebenenfalls zu einer frühzeitigen Lockerung des künstlichen Gelenks. Daraus resultiert ein vorzeitiges oder frühzeitiges Versagen des künstlichen Gelenks und eine vorzeitige Notwendigkeit zum Wechsel.

Um Gelenkknochen für den Einsatz eines künstlichen Gelenks vorzubereiten, müssen zunächst Skelettstrukturen ausgearbeitet werden, auf die anschließend die Bestandteile des künstlichen Gelenks implantiert werden können.

Zur Präparation von Skelettstrukturen werden in vielen Fällen zunächst mit Hilfe von Ausrichtungslehren Sägeblöcke positioniert, die als Schablonen für Resektionen von Knochenmaterial dienen. Entlang der durch die Sägeblöcke vorgegebenen Form wird mit Hilfe von oszillierenden Sägen ein Knochen in der Nähe des betroffenen Gelenks durchtrennt und eine oder mehrere Resektionsflächen an dem Knochen hergestellt.

Die Sägeschnitte erfolgen in vorher definierten Lagen des Gelenks, z.B. in einer Neutrallage und Mittelstellung. Anschließend werden gegebenenfalls weitere Sägelehren auf die erhaltene Knochenfläche aufgesetzt und der Knochen weiter bearbeitet, um ein gewünschtes Oberflächenprofil zu erhalten, auf das eine Probeprothese passgenau aufgesetzt werden kann, die die ehemalige Gelenkoberfläche ersetzt. Nach Abschluss der Knochenpräparation werden die aus einer speziellen Metalllegierung bestehenden Prothesenkomponenten auf die jeweiligen Schnittflächen implantiert. Zwischen den Metallkomponenten wird ein aus hochvernetztem Kunststoff stehender Gleitkern platziert.

Das auf diese Weise eingesetzte künstliche Gelenk ist nicht an die vorhandenen Bänder bzw. Ligamente, die das Gelenk halten, angepasst. Es gibt zwar über die Möglichkeit, unterschiedliche Dicken des Gleitkernes anzuwenden, noch ein gewisses Korrekturpotential. Dennoch besteht die Gefahr, dass das Gelenk unzureichend stabil ist.

Das beschriebene Verfahren wird, beispielsweise in der Kniegelenkchirurgie, dadurch verbessert, dass sogenannte Gelenkspannvorrichtungen eingesetzt werden. Eine entsprechende Gelenkspannvorrichtung ist aus WO 2008/043380 A1 bekannt. Bei dieser bekannten Gelenkspannvorrichtung werden Paare von verlängerten Armen, so genannten "Pratzen" zwischen die Knochen in das Gelenk eingeschoben. Die Pratzen werden mit einer einstellbaren Kraft auseinander gedrückt und so das Gelenk und die Ligamente des Gelenks gespannt. Mit Hilfe von solchen Gelenkspannvorrichtungen ist es möglich, die Resektion von Skelettanteilen mit einer oszillierenden Säge in einer oder mehreren unterschiedlichen Gelenkstellungen unter definierten Spannungsverhältnissen der Gelenkweichteile durchzuführen. Hieraus resultiert ein in Bezug auf die Gelenkweichteile besser ausbalanciertes Prothesengelenk. Dabei ist es möglich, die Spannungs- bzw. Abstandsverhältnisse zwischen den Prothesenkomponenten für die definierten Gelenkpositionen vorherzusagen, in denen die Knochenschnitte durchgeführt worden sind.

Zur Skelettresektion werden üblicherweise gerade Sägeblätter verwendet, so dass der fertig präparierte Skelettkörper die Form eines Polyeders darstellt.

In WO 2007/130467 A2 sind eine Vorrichtung und ein Verfahren beschrieben, bei denen eine Resektion von Skelettanteilen eines Gelenks mit Hilfe von in den Gelenkzwischenraum eingebrachten walzenartigen Fräsen bzw. einer bandschleifartigen Vorrichtung durchgeführt wird. Bei diesen Fräsen liegen die Rotationsachsen der Walzen bzw. der Bandschleifvorrichtung parallel zur resezierten Gelenkfläche.

Das Resezieren der konvexen Gelenkfläche wird gemäß WO 2007/130467 A2 unter Erzeugung einer konvexen Resektionsfläche durch mehrmaliges "Durchbewegen" des Gelenks entlang seiner relevanten Bewegungsebene durchgeführt. Ein scharnierartig bewegtes Gelenk wird dazu mehrmals um seine Gelenkachse verdreht, wobei das Gelenk durch die vorhandenen Ligamente bzw. Bänder gehalten wird. Die entstehenden Resektionsflächen sind aufgrund des Durchbewegens des Gelenks gebogene Flächen.

Die in WO 2007/130467 A2 offenbarte Fräsvorrichtung verfügt über expandierbare Einheiten, mittels derer der Druck der Fräsvorrichtung auf die Gelenkoberfläche bestimmt wird. Beim Walzfräsen bzw. bandschleifartigen Schleifen treten Kräfte auf den zu resezierenden Knorpel bzw. Knochen auf, die die gewünschte Ausrichtung beeinflussen. Beeinträchtigungen des Resektionsergebnisses werden dadurch minimiert, dass der Anpressdruck der Fräse niedrig gehalten wird. Da bei niedrigem Anpressdruck das Gelenk während des Fräsvorgangs häufig durchbewegt werden muss, bedeutet dies einen entsprechend langwierigen Operationsprozess.

WO 01/66021 A1 betrifft eine Vorrichtung mit einer Führungsschablone zur Verwendung in einer Gelenkplastik eines Kniegelenks, die dazu ausgebildet ist, das Abtragen von Gewebe des Schienbeins oder des Oberschenkelknochens zu führen, um eines der beiden mit einer Prothese auszustatten. Die Vorrichtung ist weiter ausgebildet, einen ausgewählten Abstandhalter aufzunehmen, um den Oberschenkelknochen vom Schienbein zu beabstanden, und der um das Kniegelenk herum in einer Position gehalten wird, die von dem Abstandhalter vorgegeben wird, um das Abtragen von Knochengewebe zu einer gewünschten Tiefe zu führen. Eine Oberfräse bzw. ein Plattenfräsapparat ist mit einer Schneidscheibe versehen, die sechs radial sich erstreckende Klingen aufweist, die sich jeweils von einer Schneidkante zu einer nacheilenden Kante verjüngen. Auch die äußeren peripheren Enden jedes Schneidmessers haben Schneidkanten. Die Oberfräse mit der Schneidscheibe wird auf eine im Kniegelenk angeordnete geeignet dicke Scheibe mit einer Führungsgrube für die Oberfräse eingebracht, um den Knochen zu resezieren.

In EP 1 374 783 A1 ist ein Bausatz zur Präparierung einer Knochenhöhle in einem Knochen zur Implantation einer Gelenkprothese offenbart, die eine Führungsvorrichtung mit einer Führungsöffnung aufweist, sowie ein drehbares Werkzeug, das in der Führungsöffnung der Führungsvorrichtung angeordnet ist, wobei dieses sich drehende Werkzeug ausgebildet ist, Knochen zu entfernen, um eine Knochenhöhle zu erzeugen. Für eine Präparation eines Fußgelenks ist ein Bohrer oder die Bohrerführung daran angepasst, den Bohrer in zwei verschiedenen Positionen zu halten. Dadurch kann das Fußgelenk in verschiedenen Beugungswinkeln gegenüber dem Schienbein präpariert werden.

Ausgehend von diesem Stand der Technik liegt der Erfindung die Aufgabe zugrunde, eine Vorrichtung und ein Verfahren zur In-situ-Fräsung von Gelenkflächen, insbesondere, aber nicht ausschließlich, von scharnierartig funktionierenden Gelenken, zur Verfügung zu stellen, mittels derer auf schnelle und sichere Weise Gelenkoberflächen für eine Implantation von Endoprothesen vorbereitet werden können, wobei die ursprünglich bestehende Achsenkinematik und Ligamentführung optimal wiederhergestellt werden.

Diese Aufgabe wird durch eine Vorrichtung zur In-situ-Fräsung von Gelenkflächen gelöst, umfassend eine Fräsvorrichtung und eine Gelenkspannvorrichtung, wobei die Gelenkspannvorrichtung ausgebildet ist, um zwischen zwei ein Gelenk bildende Gelenkanteile, insbesondere Knochen und/oder gelenkbildende Gleitflächen, eingebracht zu werden und die Gelenkanteile, insbesondere Knochen, unter einer vorbestimmten oder einstellbaren Spannung auseinander zu drücken, wobei die Gelenkspannvorrichtung eine Aussparung aufweist, in die die Fräsvorrichtung einbringbar ist, wobei die Fräsvorrichtung eine Stirnfräse aufweist, deren Rotationsachse im Wesentlichen senkrecht auf der zu fräsenden Fläche steht, dadurch gekennzeichnet, dass die Gelenkspannvorrichtung eine schachtförmige Aussparung aufweist, wobei die Gelenkspannvorrichtung seitlich der Aussparung Stützflächen zum Abstützen auf einem zu resezierenden Knochen aufweist, wobei die Stützflächen konkave Wölbungen zur Aufnahme gewölbter Teile des Knochens aufweisen.

Im Rahmen der Erfindung wird unter einer Stirnfräse eine Fräse verstanden, deren Rotationsachse senkrecht oder im Wesentlichen senkrecht auf der zu fräsenden Fläche steht. Die Fräsvorrichtung ist eine funktionelle Einheit.

Mit der Erfindung werden mehrere miteinander zusammenhängende Wirkungen erzielt. Eine erste Wirkung besteht in der Entkopplung der Gelenkspannvorrichtung und der Fräsvorrichtung bei der erfindungsgemäßen Vorrichtung. Aufgrund dieser Entkopplung wird beim Durchbewegen des Gelenks in jeder Gelenkposition eine durch die Gelenkspannvorrichtung vorgegebene oder einstellbare Gelenkspannung eingestellt, die einer natürlichen Spannung des Gelenks entsprechen kann, unabhängig vom Anpressdruck der Fräsvorrichtung.

Aufgrund dieser Entkopplung ist es möglich, die durch die Ligamente definierte bestehende Achsenkinematik genauer zu reproduzieren als es im Stand der Technik, etwa gemäß WO 2007/130467 A2, möglich war. Es ist erfindungsgemäß nicht mehr notwendig, einen Anpressdruck und damit die Spannung des Gelenks gering zu halten und dadurch in Kauf zu nehmen, dass durch die resultierende geringe Spannung der Ligamente die bestehende Achsenkinematik nur ungenau reproduziert wird.

Eine weitere Wirkung der Erfindung, die mit der ersten zusammenhängt, tritt aufgrund des Einsatzes einer Stirnfräse auf. Durch die Verwendung einer Stirnfräse anstelle von Walzenfräsen werden die Kräfte bzw. Scherkräfte, die beim Fräsen auf das Gelenk wirken, deutlich verringert. Dies ermöglicht eine Erhöhung des Anpressdrucks.

Da die Gelenkspannung von der Gelenkspannvorrichtung vorgegeben wird, eine Stirnfräse gegebenenfalls mit höherem Anpressdruck als bisher möglich eingesetzt werden kann und außerdem die Eindringtiefe einer Stirnfräse größer ist als die Eindringtiefe der bekannten Vorrichtung, ist es möglich, eine Resektionsfläche mit nur wenigen Durchführungen des Gelenks, vorzugsweise mit nur einer Durchführung des Gelenks, herzustellen. Die Stirnfräse kann dabei vorzugsweise so geformt sein, dass die gewünschte Tiefe der gefrästen Fläche bereits in einem Arbeitsgang erreicht wird. Hierdurch wird die Zeit, die die Operation benötigt, deutlich verkürzt.

Erfindungsgemäß weist die Gelenkspannvorrichtung, insbesondere eine als Elastomerkörper ausgebildete Gelenkspannvorrichtung, eine schachtförmige Aussparung auf, in denen eine Fräsvorrichtung eingeführt werden kann. Die Aussparung weist vorzugsweise Mittel zum Haltern, Führen und/oder Fixieren einer Fräsvorrichtung auf.

Seitlich der Aussparung weist die Gelenkspannvorrichtung, insbesondere eine als Elastomerkörper ausgebildete Gelenkspannvorrichtung, erfindungsgemäß Stützflächen zum Abstützen auf einem zu resezierenden Knochen auf. Diese Stützflächen begrenzen somit die Aussparung auf beiden Seiten. Insbesondere in dem Fall, wenn der weitere zu resezierende Knochen eine konvexe Gelenkfläche aufweist, weisen die Stützflächen konkave Wölbungen zur Aufnahme gewölbter Teile des Knochens auf. Die konkaven Wölbungen der Stützflächen dienen somit der Führung des weiteren zu resezierenden Knochens bzw. der weiteren zu resezierenden Gelenkanteile während des Durchführens des Gelenks und hindern den Knochen bzw. den Gelenkkörper daran, während des Fräsens aus der Position zu geraten.

Vorzugsweise weist die Stirnfräse sowohl stirnseitig als auch an ihrem Umfang Fräsflächen auf. Weiter vorzugsweise weist die Stirnfräse ein gewölbtes Fräsprofil auf. Auf diese Weise ist es möglich, mit der Stirnfräse durch seitlichen Vorschub der Fräsvorrichtung, also einen Vorschub quer zur Rotationsachse der Stirnfräse, bzw. mittels gelenkiger Bewegung des Gelenks in einem Zug eine tiefe Fräsung bzw. Resektionsfläche zu erzeugen.

Vorteilhafterweise weist die Fräsvorrichtung ein Chassis für die Stirnfräse auf, das in die Aussparung der Gelenkspannvorrichtung einführbar und/oder in der Aussparung der Gelenkspannvorrichtung fixierbar ist. Das Chassis weist vorzugsweise die Ausmaße der Aussparung auf, so dass es rutsch- bzw. verschiebesicher im Gelenk angeordnet werden kann.

In einer vorteilhaften Weiterbildung ist die Fräsvorrichtung mittels eines Zahnradantriebs mit Zahnrädern antreibbar, wobei ein Zahnrad mit der Stirnfräse verbunden ist oder einstückig mit der Stirnfräse ausgebildet ist und ein anderes Zahnrad, das in das mit der Stirnfräse verbundene oder mit der Stirnfräse einstückig ausgebildete Zahnrad eingreift, mit einer Welle verbunden ist. Die Welle ist vorzugsweise mit einer Antriebsvorrichtung verbunden. Die Antriebsvorrichtung kann ein Handantrieb oder ein elektrischer Antrieb sein, wobei die Antriebsgeschwindigkeit insbesondere vorteilhafterweise regelbar ist. Mittels des Zahnradantriebs ist es möglich, die Stirnfräse mit senkrecht auf der Gelenkfläche stehender Rotationsachse platzsparend und effektiv anzutreiben.

Die Gelenkspannvorrichtung ist vorzugsweise hydraulisch ausgebildet und/oder mit Pratzen ausgebildet, die im in ein Gelenk eingeführten Zustand auf Gelenkteilen aufliegen. Eine Pratzen verwendende Vorrichtung ist etwa aus WO 2008/043380 A1 bekannt.

Alternativ oder zusätzlich hierzu ist in einer bevorzugten Ausführungsform die Gelenkspannvorrichtung als ein- oder mehrstückiger Elastomerkörper ausgebildet oder umfasst einen oder mehrere Elastomerkörper. Durch einen Elastomerkörper wird das Gelenk entsprechend der Gelenkspannung ausgerichtet. So lässt sich in jeder Gelenkstellung ein definierter Druck erzeugen, der unabhängig vom Anpressdruck der Fräsvorrichtung ist. Mehrere Elastomerstücke oder Elastomerkörper können als Auflageflächen für Pratzen dienen.

Die Gelenkspannvorrichtung, insbesondere der Elastomerkörper, weist vorzugsweise eine Auflagefläche für eine Resektionsfläche eines Knochens auf. Damit ist der Elastomerkörper bzw. die Gelenkspannvorrichtung in den vergrößerten Zwischenraum einführbar und an die Resektionsfläche des Knochens anlegbar, der bereits eine beispielsweise flache Resektionsfläche aufweist.

Vorteilhafterweise umfasst die Vorrichtung eine Basiseinheit, die an einem Knochen fixierbar ist und Mittel zur Halterung der Gelenkspannvorrichtung aufweist. Eine solche Basiseinheit kann auch zur Halterung von Sägeschablonen u. a. dienen. Insbesondere weist die Gelenkspannvorrichtung, insbesondere der Elastomerkörper, vorteilhafterweise Öffnungen oder Mittel zur Halterung an der Basiseinheit und/oder zum Eingriff mit Haltemitteln der Basiseinheit auf.

Die vorstehend beschriebene erfindungsgemäße Vorrichtung kann vorteilhaft zur In-situ-Fräsung von Gelenkflächen verwendet werden.

Ebenfalls können in einem Verfahren zur In-situ-Fräsung von Gelenkflächen mittels einer vorstehend beschriebenen erfindungsgemäßen Vorrichtung die folgenden Verfahrensschritte ausgeführt werden:
- Einschieben einer Gelenkspannvorrichtung zwischen die an das Gelenk angrenzenden Knochen bzw. Gelenkanteile, von denen ein Knochen bzw. Gelenkanteil bereits reseziert ist, wobei das Gelenk unter eine vorbestimmte oder einstellbare Spannung gesetzt wird,
- Einbringen einer Fräsvorrichtung mit einer Stirnfräse in eine Schachtaussparung der Gelenkspannvorrichtung, wobei insbesondere die Stirnfräse während des Einbringens angetrieben wird, um eine Resektionsfläche beim noch nicht resezierten Knochen bzw. Gelenkanteil zu bilden,
- Herstellen einer gekrümmten Resektionsfläche am noch nicht resezierten Knochen bzw. Gelenkanteil mittels, insbesondere einmaligen, Durchbewegens des Gelenks.

Auf diese Weise ist eine gewölbte Resektionsfläche sehr schnell und unter Behalt der ursprünglich bestehenden Achsenkinematik und Ligamentführung des Gelenks herstellbar.

Vorteilhafterweise wird zuvor, insbesondere unter Anbringung einer Basiseinheit an einem an ein Gelenk angrenzenden Knochen und Aufbringen einer Sägelehre auf die Basiseinheit, eine Resektionsfläche an dem Knochen hergestellt.

Weiter vorzugsweise wird im Anschluss an die Herstellung einer Resektionsfläche an dem Knochen die Sägelehre von der Basiseinheit entfernt.

Schließlich werden vorzugsweise im Anschluss an die Herstellung einer gekrümmten Resektionsfläche die Fräsvorrichtung und die Gelenkspannvorrichtung aus dem Gelenk entfernt. Es können dann seitliche Knochenreste, auf denen die Gelenkspannvorrichtung zuvor aufgelegen hatte, entfernt werden, um eine endgültige gekrümmte Resektionsfläche zu erhalten. Bei bestimmten Gelenktypen (z.B. oberes Sprunggelenk) werden die Gelenkflächen, auf denen die Gelenkspannvorrichtung zuvor aufgelegen hatte, entfernt, um den entsprechenden Gelenkanteil vollständig mit der Prothese überdecken zu können.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Rückansicht eines oberen Sprunggelenks mit Basiseinheit,
- Fig. 2: das Ensemble gemäß Fig. 1 in schematischer Seitenansicht,
- Fig. 3: eine schematische Rückansicht gemäß Fig. 1 mit auf der Basiseinheit aufgesetzter Sägelehre,
- Fig. 4: das Ensemble gemäß Fig. 3 in schematischer Seitenansicht,
- Fig. 5: eine schematische Rückansicht gemäß Fig. 3 mit auf die Basiseinheit aufgeschobener Gelenkspannvorrichtung,
- Fig. 6: das Ensemble gemäß Fig. 5 in schematischer Seitenansicht,
- Fig. 7: eine schematische Rückansicht gemäß Fig. 3 mit einer erfindungsgemäßen Vorrichtung mit Fräsvorrichtung vor dem Einschub in eine Gelenkspannvorrichtung,
- Fig. 8: das Ensemble gemäß Fig. 7 in schematischer Seitenansicht,
- Fig. 9: eine Ansicht gemäß Fig. 8 mit eingeschobener Fräsvorrichtung in Mittelstellung des oberen Sprunggelenks,
- Fig. 10: eine Ansicht gemäß Fig. 9 mit durchgeführtem oberen Sprunggelenk,
- Fig. 11: eine schematische Rückansicht des teilweise resezierten Sprungbeins und Schienbeins,
- Fig. 12: das Ensemble gemäß Fig. 11 in schematischer Seitenansicht,
- Fig. 13: das Ensemble gemäß Fig. 11 mit eingelegter gekrümmter Sägelehre,
- Fig. 14: eine schematische Rückansicht eines oberen Sprunggelenks mit vollständig präparierten Resektionsflächen und
- Fig. 15: das Ensemble gemäß Fig. 14 in schematischer Seitenansicht.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

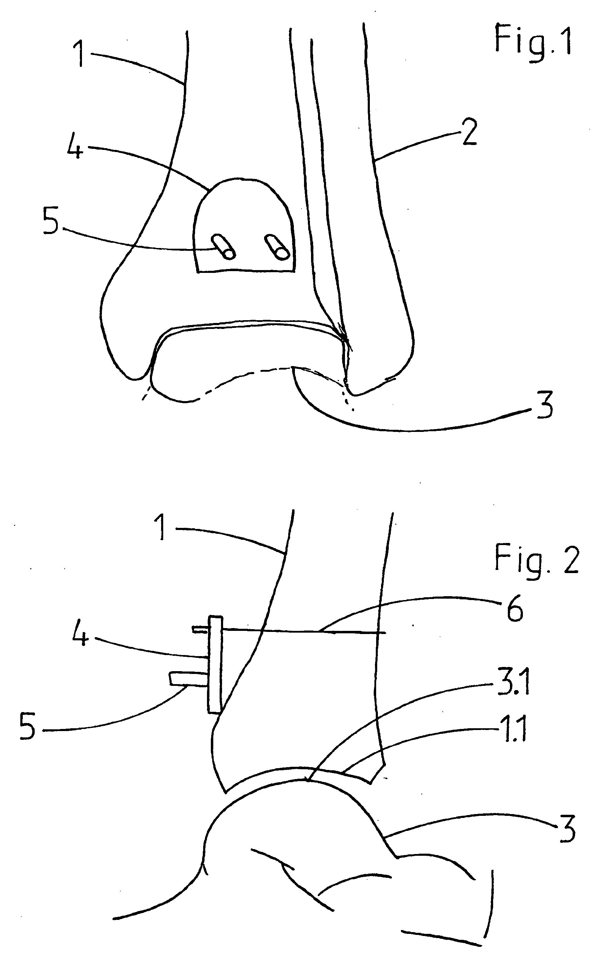

Die Fig. 1 bis 15 zeigen jeweils in stark schematisierter Darstellung den Ablauf einer beidseitigen Gelenkresektion am Beispiel eines oberen Sprunggelenks. Fig. 1 zeigt eine schematische Rückansicht eines oberen Sprunggelenks mit Schienbein 1 (Tibia), Wadenbein 2 (Fibula) und Sprungbein 3 (Talus). Das Sprungbein 3 ist in Fig. 1 und den folgenden Figuren nur anhand des Gelenkkopfs teilweise dargestellt.

Auf der Rückseite des Schienbeins 1 ist eine Basiseinheit 4 fixiert, die Bolzen 5 als Mittel zur Halterung von Sägelehren, Gelenkspannvorrichtungen etc. aufweist, die im Folgenden noch dargestellt werden.

Fig. 2 zeigt eine schematische Seitendarstellung der Darstellung gemäß Fig. 1. Zu erkennen ist zusätzlich eine Halterung 6, mittels der die Basiseinheit 4 im Schienbein 1 verschraubt oder verankert ist. Die Basiseinheit 4 ist fest mit dem Schienbein 1 verbunden und bietet somit einen gleichbleibenden Referenzpunkt für die nachfolgenden Schnitte und Fräsbewegungen. Weiterhin sind eine konkave Gelenkfläche 1.1 des Schienbeins 1 und eine konvexe Gelenkfläche 3.1 des Sprungbeins 3 dargestellt.

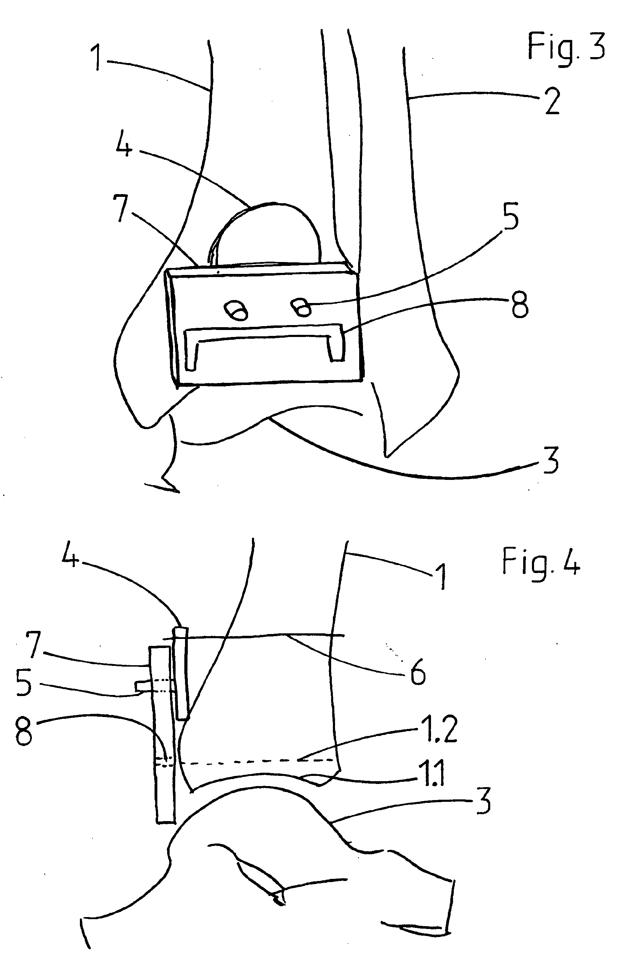

Im nächsten Schritt, der in Fig. 3 dargestellt ist, ist eine Sägelehre 7 auf die Bolzen 5 der Basiseinheit 4 aufgesteckt worden. Die Sägelehre 7 weist einen umgekehrt U-förmigen Schlitz 8 auf, der eine Resektionsfläche in mit drei Abschnitten für das Schienbein 1 definiert. Seitliche Stücke des Schienbeins 1 bleiben bei der Resektion dadurch erhalten.

Durch den Schlitz 8 wird ein nicht dargestelltes oszillierendes Sägeblatt geschoben, um einen entsprechenden Schnitt zu vollziehen und eine Resektionsfläche 1.2 herzustellen.

Die geplante Resektionsfläche 1.2 am distalen Ende des Schienbeins 1 ist in gestrichelter Weise in Fig. 4 in seitlicher Darstellung angedeutet. Fig. 4 zeigt eine Schnittdarstellung senkrecht zur Sichtfläche aus Fig. 3, die etwa durch den Bolzen 5 aus Fig. 3 verläuft, der in Fig. 4 ebenfalls dargestellt ist. Deutlich sichtbar ist, dass gegenüber der konkaven Gelenkfläche 1.1 die Resektionsfläche 1.2 am Schienbein 1 zurückversetzt und gerade bzw. flach ist.

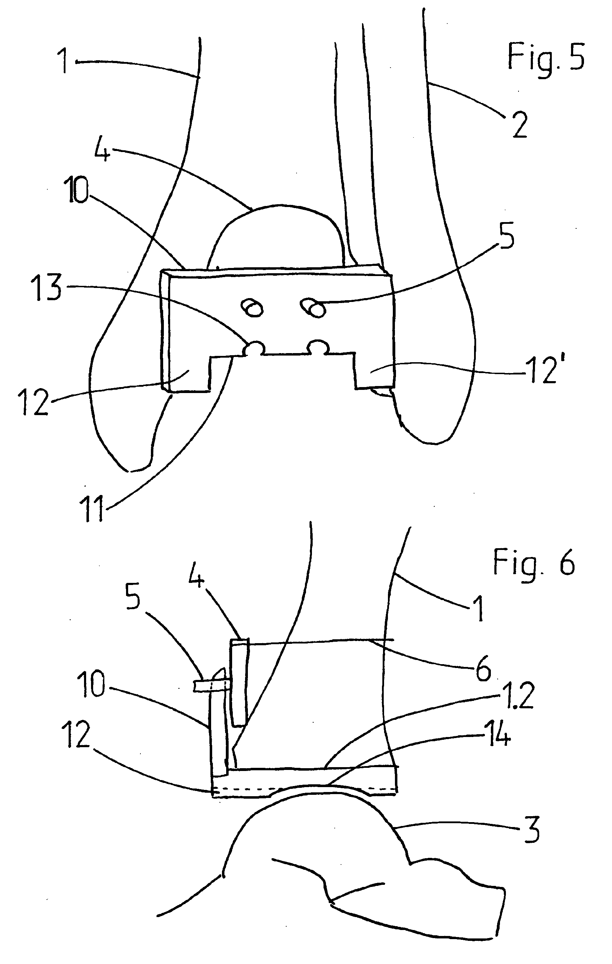

Nach erfolgter Herstellung einer Resektionsfläche 1.2 am Schienbein 1 wird die Sägelehre 7 abgenommen und durch eine Gelenkspannvorrichtung 10 ersetzt, der in Fig. 5 bis 10 durch einen Elastomerkörper verkörpert ist. Die Rückansicht, die in Fig. 5 schematisch dargestellt ist, zeigt, dass die Gelenkspannvorrichtung 10 eine Schachtaussparung 11 aufweist, in die eine Fräsvorrichtung einführbar ist, sowie in der oberen Begrenzungsfläche der Schachtaussparung 11 Führungsnuten 13 für eine Fräsvorrichtung. Seitlich wird die Schachtaussparung 11 durch Stützflächen 12, 12' begrenzt, die auf dem Sprungbein 3 aufliegen, das in Fig. 5 nicht gezeigt ist.

In der Seitenansicht gemäß Fig. 6, die zu Fig. 5 korrespondiert, ist zu erkennen, dass die Stützfläche 12 der Gelenkspannvorrichtung 10 eine konkave Wölbung 14 aufweist, die mit der Oberfläche des Sprungbeins 3 zusammenwirkt. Die konkave Wölbung 14 verhindert, dass das Sprungbein 3 während der Resektion nach vorne oder nach hinten verrutschen kann, erlaubt aber ein Durchführen des oberen Sprunggelenks, also eine Verdrehung des Sprungbeins 3 gegenüber dem Schienbein 1. Dabei bietet die konkave Wölbung ein Widerlager für das Sprungbein 3 während der Durchführung des Gelenks während des Fräsvorgangs.

Ebenfalls ist in Fig. 6 zu erkennen, dass die Resektionsfläche 1.2 am distalen, d. h. dem körperabgewandten, Ende des Schienbeins als Auflagefläche für den in das Gelenk eingeschobenen Teil der Gelenkspannvorrichtung 10 dient. Mit der Gelenkspannvorrichtung 10 wird erreicht, dass der Elastomerkörper 10 sich lediglich auf den äußeren bzw. inneren Anteil der Gelenkoberfläche des Sprungbeins abstützt, d.h. auf seinen Schultern. Dazwischen wird ein definierter Zwischenraum freigelassen, die Schachtaussparung 11, die als Arbeitsschacht dient.

Durch die Elastomere wird das Sprungbein entsprechend der natürlichen Gelenkspannung ausgerichtet, und zwar in jeder Lage des oberen Sprunggelenks. Hierzu wirkt der Elastomerkörper 10 mit den Ligamenten bzw. Bändern des Sprungbeins zusammen. Die Spannung des Elastomerkörpers ist vorzugsweise so konzipiert, dass das Gelenk bis zu einer gewissen Maximalspannung ausgerichtet wird, das Material beim Durchbewegen aber nachgibt, falls die Gelenkspannung einen bestimmten Wert überschreitet.

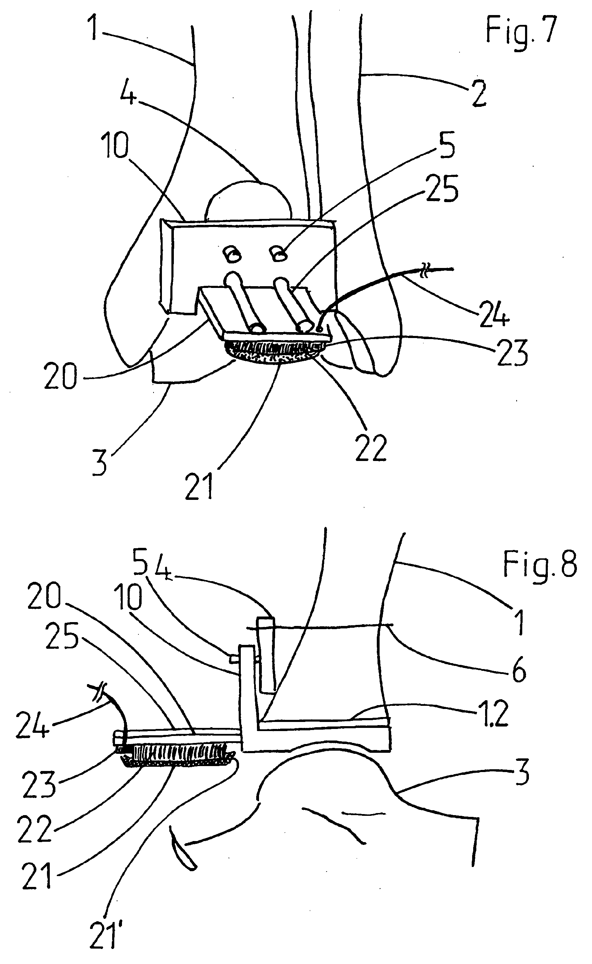

In Fig. 7 ist in einer schematischen Rückansicht dargestellt, dass eine Fräsvorrichtung im Begriff ist, in die Aussparung 11 der Gelenkspannvorrichtung 10 eingeschoben zu werden. Die Fräsvorrichtung weist ein Chassis 20 auf, das die gleiche Breite wie die Aussparung 11 hat. Am Chassis 20 sind auf der der Gelenkspannvorrichtung 10 zugewandten Seite Führungskörper 25 vorgesehen, die in die Führungsnuten 13 der Gelenkspannvorrichtung 10 eingreifen und mittels derer die Fräsvorrichtung in der Gelenkspannvorrichtung 10 gehaltert, geführt und/oder fixiert wird.

Auf der dem Sprungbein 3 zugewandten Seite weist die Fräsvorrichtung eine Stirnfräse 21 auf sowie zwischen dem Chassis 20 und der Stirnfräse 21 ein Zahnrad 22, das auf der gleichen Achse wie die Stirnfräse 21 rotierbar angeordnet ist. Das Zahnrad 22 ist beispielsweise einstückig mit der Stirnfräse 21 ausgebildet. Ein kleineres Zahnrad 23 ist ebenfalls am Chassis 20 angeordnet und greift in das Zahnrad 22 der Stirnfräse 21 ein. Das Zahnrad 23 wird mittels einer Welle 24 von einer nicht dargestellten Antriebsvorrichtung angetrieben. Die Rotationsachse des Zahnrads 23 kann parallel zur Rotationsachse der Stirnfräse 21 und des Zahnrads 22 sein oder in einem Winkel dazu angeordnet sein, etwa einem rechten bzw. 90°- Winkel. In den Fig. 7 bis 10 ist auch jeweils die Zahnrändelung der Zahnräder 22, 23 dargestellt.

Fig. 8 zeigt eine schematische Seitenansicht der Ansicht gemäß Fig. 7. Es ist zu erkennen, dass die Stirnfräse 21 sowohl eine stirnseitige Fräsfläche als auch abgewinkelte seitliche Fräsflächen aufweist.

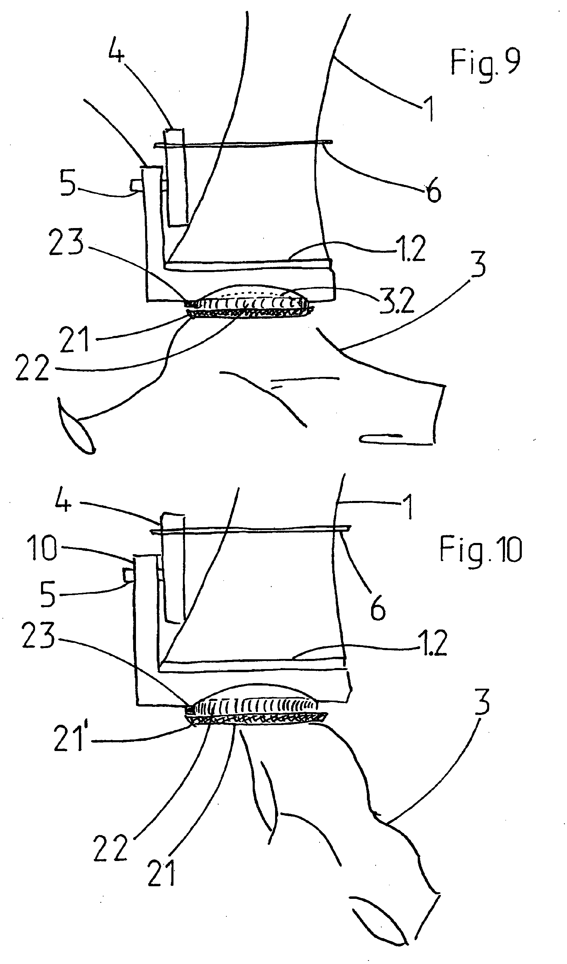

In den Fig. 9 und 10 ist die Fräsvorrichtung in dem in die Gelenkspannvorrichtung 10 eingeführten Zustand dargestellt. Bereits beim Einführen der Fräsvorrichtung kann die Stirnfräse angetrieben sein und einen ersten Teil einer Resektionsfläche erzeugen. Es handelt sich bei Fig. 9 und 10 um Schnittdarstellungen durch eine senkrechte Ebene durch das obere Sprunggelenk, etwa in der Höhe der Bolzen 5. In Fig. 9 ist das obere Sprunggelenk in einer neutralen Mittelstellung dargestellt, in Fig. 10 ist das obere Sprunggelenk, das mit einem nur angedeuteten Sprungbein 3 dargestellt ist, in eine Extremposition gebracht worden. In diesem Zustand wird das obere Sprunggelenk durch den Elastomerkörper 10 unter Spannung gesetzt und durch die Sprunggelenksligamente gehalten, so dass das Durchführen des Gelenks dem natürlichen Bewegungsablauf entspricht. Die dabei entstehende untere Resektionsfläche 3.3 am Sprungbein 3 entspricht somit ideal dem natürlichen Bewegungsablauf des oberen Sprunggelenks. Die Resektionsfläche 3.3 wird dabei sowohl durch die Stirnfräse 21 als auch durch die seitliche Fräsfläche 21' der Stirnfräse 21 erzeugt. Alternativ ist die Stirnfräse 21 leicht gewölbt ausgebildet.

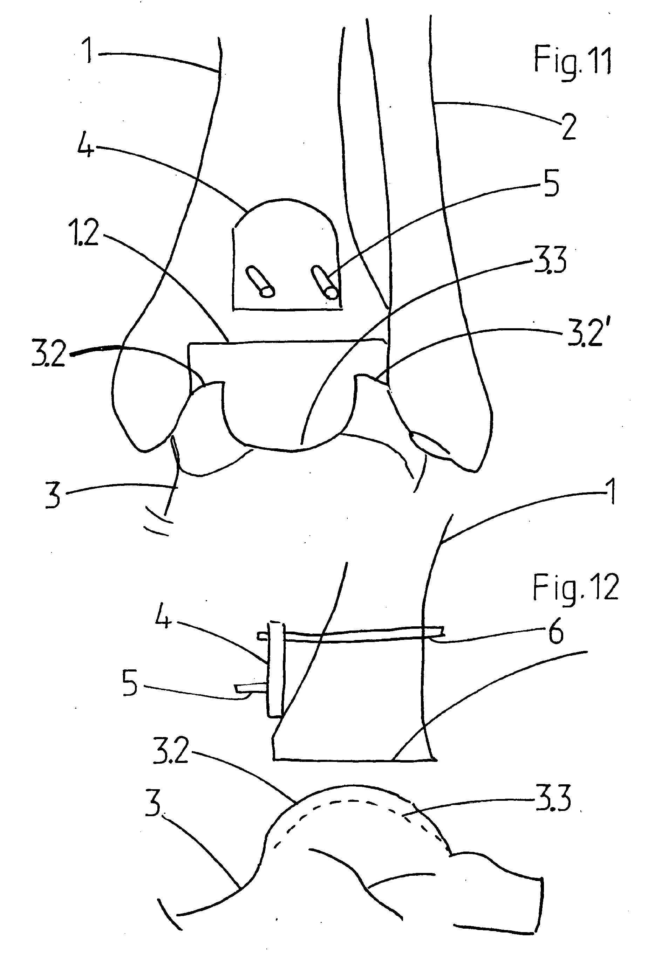

Fig. 11 und 12 zeigen in schematischer Rück- und Seitenansicht das teilresezierte obere Sprunggelenk nach Entnahme der Gelenkspannvorrichtung 10. Das Sprungbein 3 weist eine zentrale temporäre Resektionsfläche 3.3 am Sprungbein 3 auf, die gewölbt ist. Beiderseits der temporären Resektionsfläche 3.3 befinden sich seitliche Stützbogen 3.2, 3.2', auf denen das Schienbein 1 bzw. das Wadenbein 2 aufliegen. In Fig. 12 ist die temporäre Resektionsfläche 3.3 gestrichelt dargestellt, der seitliche Stützbogen 3.2 mit durchgezogenem Strich.

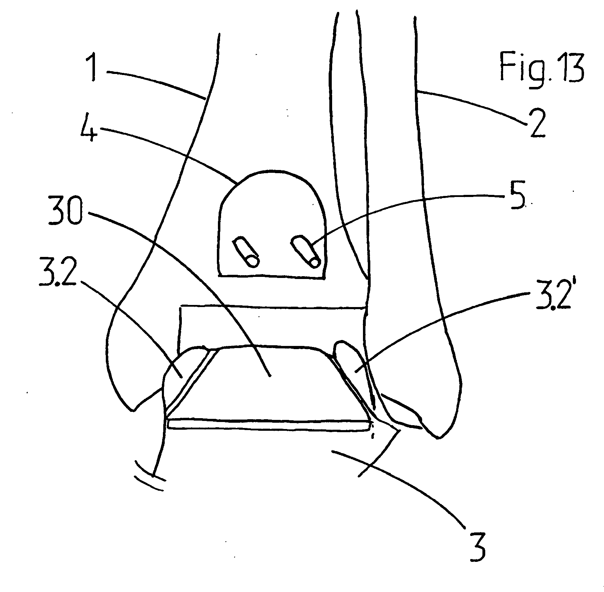

Fig. 13 zeigt in schematischer Rückansicht des oberen Sprunggelenks, dass auf die temporäre Resektionsfläche 3.3 am Sprungbein 3 eine gekrümmte Sägelehre 30 aufgesetzt ist, die in der Krümmung an die Resektionsfläche 3.3 angepasst ist. Die Sägelehre dient dazu, eine Säge zu führen, mittels der die seitlichen Stützbögen 3.2, 3.2' entfernt werden.

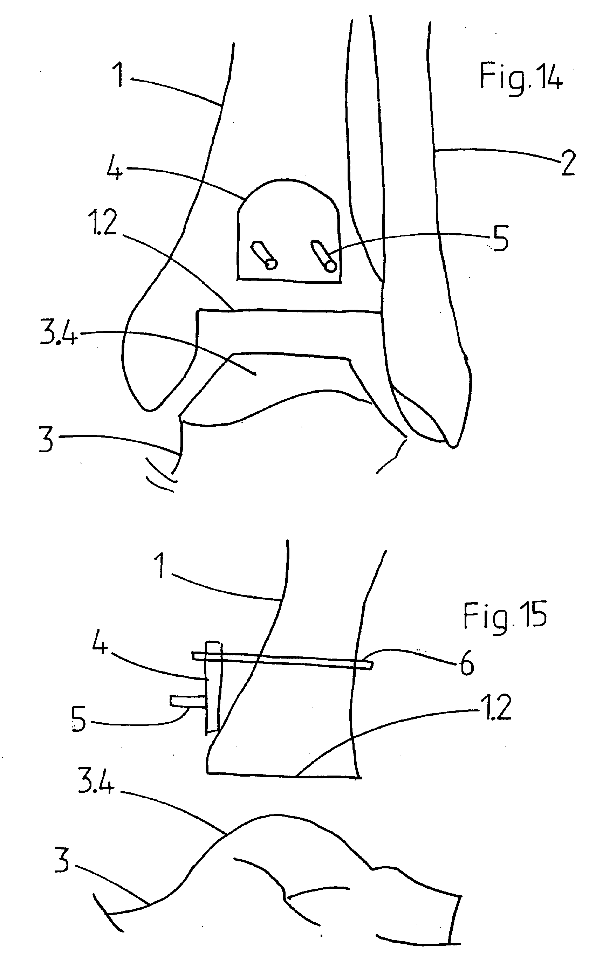

Das Ergebnis des anschließenden Entfernens der seitlichen Stützbögen ist in Fig. 14 und Fig. 15 in schematischer Rück- und Seitenansicht dargestellt. Das Sprungbein 3 weist nunmehr eine vollständige Resektionsfläche 3.4 auf. Schienbein 1 und Wadenbein 2 liegen nicht mehr auf dem Sprungbein 3 auf, so dass das obere Sprunggelenk nunmehr so vorbereitet ist, dass Endoprothesen implantiert werden können, die die ursprünglichen Gelenkflächen ersetzen. Da die Erzeugung der Resektionsfläche 3.4 am Sprungbein 3 unter Durchführung des Gelenks bei einer vorbestimmten Spannung erzeugt worden ist, die einer natürlichen Spannung der Ligamente des oberen Sprunggelenks entspricht, entspricht die erzeugte Resektionsfläche 3.4 der natürlichen, durch die Ligamente festgelegten Bewegungsmuster. Auch die Endoprothesen können nach ihrer Implantation diese Form aufweisen.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Schienbein (Tibia)
- 1.1: konkave Gelenkfläche
- 1.2: Resektionsfläche am distalen Ende des Schienbeins
- 2: Wadenbein (Fibula)
- 3: Sprungbein (Talus)
- 3.1: konvexe Gelenkfläche
- 3.2, 3.2': seitlicher Stützbogen
- 3.3: temporäre Resektionsfläche am Sprungbein
- 3.4: vollständige Resektionsfläche am Sprungbein
- 4: Basiseinheit
- 5: Bolzen
- 6: Halterung
- 7: Sägelehre
- 8: Schlitz
- 10: Gelenkspannvorrichtung
- 11: Schachtaussparung
- 12, 12': Stützfläche
- 13: Führungsnut
- 14: konkave Wölbung
- 20: Chassis
- 21: Stirnfräse
- 21': seitliche Fräsfläche
- 22: Zahnrad
- 23: Zahnrad
- 24: Antriebswelle
- 25: Führungskörper
- 30: gekrümmte Sägelehre

## Patentansprüche

1. Vorrichtung zur In-situ-Fräsung von Gelenkflächen, umfassend eine Fräsvorrichtung (20 - 24) und eine Gelenkspannvorrichtung (10), wobei die Gelenkspannvorrichtung (10) ausgebildet ist, um zwischen zwei ein Gelenk bildende Gelenkanteile (1, 3), insbesondere Knochen, eingebracht zu werden und die Gelenkanteile unter einer vorbestimmten oder einstellbaren Spannung auseinander zu drücken, wobei die Gelenkspannvorrichtung (10) eine Aussparung (11) aufweist, in die die Fräsvorrichtung (20 - 24) einbringbar ist, wobei die Fräsvorrichtung (20 - 24) eine Stirnfräse (21) aufweist, deren Rotationsachse im Wesentlichen senkrecht auf der zu fräsenden Fläche steht, wobei die Aussparung (11) eine schachtförmige Aussparung (11) ist, und wobei die Gelenkspannvorrichtung (10) seitlich der Aussparung (11) Stützflächen (12, 12') zum Abstützen auf einem zu resezierenden Knochen (3) aufweist,
**dadurch gekennzeichnet, dass** die Stützflächen (12, 12') konkave Wölbungen (14) zur Aufnahme gewölbter Teile des Knochens (3) aufweisen.

2. Vorrichtung nach Anspruch 1, **dadurch gekennzeichnet, dass** die Stirnfräse (21) sowohl stirnseitig als auch an ihrem Umfang Fräsflächen aufweist oder ein gewölbtes Fräsprofil aufweist.

3. Vorrichtung nach Anspruch 1 oder 2, **dadurch gekennzeichnet, dass** die Fräsvorrichtung (20 - 24) ein Chassis (20) für die Stirnfräse (21) aufweist, das in die Aussparung (11) der Gelenkspannvorrichtung (10) einführbar und/oder in der Aussparung (11) der Gelenkspannvorrichtung (10) fixierbar ist.

4. Vorrichtung nach einem der Ansprüche 1 bis 3, **dadurch gekennzeichnet, dass** die Fräsvorrichtung (20 - 24) mittels eines Zahnradantriebs mit Zahnrädern (22, 23) antreibbar ist, wobei ein Zahnrad (22) mit der Stirnfräse (21) verbunden ist oder einstückig mit der Stirnfräse (21) ausgebildet ist und ein anderes Zahnrad (23), das in das mit der Stirnfräse (21) verbundene oder einstückig mit der Stirnfräse (21) ausgebildete Zahnrad (22) eingreift, mit einer Welle (24) verbunden ist, wobei insbesondere die Welle (24) mit einer Antriebsvorrichtung verbunden ist.

5. Vorrichtung nach einem der Ansprüche 1 bis 4, **dadurch gekennzeichnet, dass** die Gelenkspannvorrichtung (10) hydraulisch ausgebildet ist.

6. Vorrichtung nach einem der Ansprüche 1 bis 5, **dadurch gekennzeichnet, dass** die Gelenkspannvorrichtung (10) mit Pratzen ausgebildet ist, die im in ein Gelenk eingeführten Zustand auf Gelenkteilen aufliegen.

7. Vorrichtung nach einem der Ansprüche 1 bis 6, **dadurch gekennzeichnet, dass** die Gelenkspannvorrichtung (10) als ein- oder mehrstückiger Elastomerkörper ausgebildet ist oder einen oder mehrere Elastomerkörper umfasst.

8. Vorrichtung nach einem der Ansprüche 1 bis 7, **dadurch gekennzeichnet, dass** die Gelenkspannvorrichtung (10) eine Auflagefläche für eine Resektionsfläche (1.2) eines Knochens (1) aufweist.

9. Vorrichtung nach einem der Ansprüche 1 bis 8, **dadurch gekennzeichnet, dass** die Aussparung (11) Mittel (13) zum Haltern, Führen und/oder Fixieren einer Fräsvorrichtung (20 - 24) aufweist.

10. Vorrichtung nach einem der Ansprüche 1 bis 9, **dadurch gekennzeichnet, dass** die Vorrichtung eine Basiseinheit (4) umfasst, die an einem Knochen (1) fixierbar ist und Mittel (5) zur Halterung der Gelenkspannvorrichtung (10) aufweist, wobei insbesondere die Gelenkspannvorrichtung (10) Öffnungen oder Mittel zur Halterung an der Basiseinheit (4) und/oder zum Eingriff mit Haltemitteln (5) der Basiseinheit (4) aufweist.

## Claims

1. Apparatus for the in-situ milling of joint surfaces, including a milling device (20 - 24) and a joint stretching device (10), wherein the joint stretching device (10) is constructed to be inserted between two joint portions (1, 3), particularly bones, forming a joint and to force the joint portions apart under a predetermined or adjustable pressure, wherein the joint stretching device (10) has a recess (11), into which the milling device (20 - 24) may be inserted, wherein the milling device (20 - 24) has a face milling cutter (21), the axis of rotation of which extends substantially perpendicularly to the surface to be milled, wherein the recess (11) is a shaft-shaped recess (11) and wherein the joint stretching device (10) has support surfaces (12, 12') laterally of the recess (11) for bracing on a bone (3) to be re-sectioned, **characterised in that** the support surfaces (12, 12') have concavities (14) for receiving domed portions of the bone (3).

2. Apparatus as claimed in Claim 1, **characterised in that** the face milling device (21) has milling surfaces or a domed milling profile both on its end and also on its periphery.

3. Apparatus as claimed in Claim 1 or 2, **characterised in that** the milling device (20 - 24) has a chassis (20) for the surface milling device which may be introduced into the recess (11) in the joint stretching device (10) and/or may be fixed in position in the recess (11) in the joint stretching device (10).

4. Apparatus as claimed in one of Claims 1 to 3, **characterised in that** the milling device (20 - 24) may be driven by means of a gear drive with gearwheels (22, 23), wherein a gearwheel (24) is connected to the face milling device (21) or is formed integrally with the face milling device (21) and another gearwheel (23), which is in engagement with the gearwheel (22) connected to the face milling device (21) or constructed integrally with the face milling device (21), with which a shaft (24) is connected, whereby, in particular, the shaft (24) is connected to an actuator.

5. Apparatus as claimed in one of Claims 1 to 4, **characterised in that** the joint stretching device (10) is of hydraulic construction.

6. Apparatus as claimed in one of Claims 1 to 5, **characterised in that** the joint stretching device (10) is constructed with lugs which rest on portions of the joint in the state in which it is inserted into a joint.

7. Apparatus as claimed in one of Claims 1 to 6, **characterised in that** the joint stretching device (10) is constructed in the form of a single- or multiple-piece elastomer body or includes one or more elastomer bodies.

8. Apparatus as claimed in one of Claims 1 to 7, **characterised in that** the joint stretching device (10) has an engagement surface for a resection surface (1.2) of **a** bone (1).

9. Apparatus as claimed in one of Claims 1 to 8, **characterised in that** the recess (11) has means (13) for holding, guiding and/or fixing a milling device (20 - 24).

10. Apparatus as claimed in one of Claims 1 to 9, **characterised in that** the apparatus includes a base unit (4), which is fixable to a bone (1) and has means (5) for mounting the joint stretching device (10), wherein, in particular, the joint stretching device (10) has openings or means for holding onto the base unit (14) and/or for engaging with retaining means (5) on the base unit (4).

## Revendications

1. Dispositif pour le fraisage in situ de surfaces articulaires, comprenant un dispositif de fraisage (20 à 24) et un dispositif de mise en tension des articulations (10), dans lequel le dispositif de mise en tension des articulations (10) est adapté entre deux parties formant l'articulation (1, 3), en particulier entre les os, pour soumettre les parties de l'articulation à une tension prédéterminée ou à une tension réglable, le dispositif de mise en tension des articulations (10) présentant un évidement (11), dans lequel le dispositif de fraisage (20 à 24) peut être inséré, dispositif dans lequel le dispositif de fraisage (20 à 24) comprend une fraise à queue (21) dont l'axe de rotation est sensiblement perpendiculaire aux surfaces à fraiser, ledit évidement (11) présentant un évidement en forme d'arbre (11), dans lequel le dispositif de mise en tension des articulations (10) est agencé sur le côté de l'évidement (11), **caractérisé en ce que** le dispositif comprend des surfaces d'appui (12, 12') pour supporter un os (3) devant être réséqué, les surfaces d'appui (12, 12') présentant des dépressions concaves (14) de manière à recevoir les parties courbées de l'os (3).

2. Dispositif selon la revendication 1, **caractérisé en ce que** la fraise à queue (21) présente deux arrêtes arrondies sur sa surface supérieure ainsi qu'une surface de coupe incurvée au niveau de sa surface de fraisage.

3. Appareil selon la revendication 1 ou la revendication 2, **caractérisé en ce que** le dispositif de fraisage (20 à 24) comprend un châssis (20) afin de recevoir la fraise à queue (21) dans l'évidement (11) du dispositif de mise en tension des articulations (10), la fraise à queue étant insérée et / ou fixée dans l'évidement (11) du dispositif de mise en tension des articulations (10).

4. Dispositif selon l'une quelconque des revendications 1 à 3, **caractérisé en ce que** le dispositif de fraisage (20 à 24) peut être entraîné par l'intermédiaire d'un engrenage à roues dentées (22, 23), dans lequel un engrenage (22) est relié à la fraise à queue (21) ou fait partie intégrante de la fraise à queue (21), l'engrenage venant en prise avec un autre engrenage (23) associé à la fraise à queue (21) ou solidaire de la fraise à queue (21), l'engrenage (23) étant relié à un arbre (24), en particulier dispositif dans lequel l'arbre (24) est relié à un dispositif d'entraînement.

5. Dispositif selon l'une quelconque des revendications 1 à 4, **caractérisé en ce que** le dispositif de mise en tension des articulations (10) est un dispositif hydraulique.

6. Dispositif selon l'une quelconque des revendications 1 à 5, **caractérisé en ce que** le dispositif de mise en tension des articulations (10) comprend des extrémités qui sont insérées autour d'une articulation.

7. Dispositif selon l'une quelconque des revendications 1 à 6, **caractérisé en ce que** le dispositif de mise en tension des articulations (10) est un ensemble de plusieurs pièces en élastomère ou comprend un ou plusieurs ensembles en élastomère.

8. Dispositif selon l'une quelconque des revendications 1 à 7, **caractérisé en ce que** le dispositif de mise en tension des articulations (10) présente une surface d'appui pour la résection d'une surface (1, 2) d'un os (1).

9. Dispositif selon l'une quelconque des revendications 1 à 8, **caractérisé en ce qu'**il comprend un évidement (11), des supports (13), des cavités et/ou des moyens de fixations d'un dispositif de fraisage (20 à 24).

10. Dispositif selon l'une quelconque des revendications 1 à 9, **caractérisé en ce que** le dispositif comprend un corps de base (4) fixé sur un os (1) et des moyens de retenue (5) pour maintenir le dispositif de mise en tension des articulations (10), dispositif comprenant en particulier des ouvertures dans ledit dispositif de mise en tension des articulations (10) ou des moyens pour le monter sur le corps de base (4) et / ou pour qu'il vienne en prise avec les moyens de retenue (5) du corps de base (4).
